# EUROPEAN PATENT SPECIFICATION

(11) **EP 0 519 765 B1**
(45) Date of publication and mention of the grant of the patent: **08.10.1997**
(21) Application number: 92305732.7
(22) Date of filing: 22.06.1992
(51) Int. Cl.: A61M 5/142, A61M 5/168

(54) **Implantable medication infusion pump including self-contained acoustic fault detection apparatus**
Implantierbare Arzneimittelinfusionspumpe mit akustischer Fehlerdetektionsvorrichtung
Pompe implantable pour perfusion de produits pharmaceutique avec dispositif acoustique de détection de défauts

(30) Priority: 21.06.1991 US 719221; 21.06.1991 US 718709
(43) Date of publication of application: 23.12.1992
(73) Proprietor: Minimed Inc., doing business as Minimed Technologies, Sylmar, California 91242 (US)
(72) Inventor: Lord, Peter C., Valencia, CA 91355 (US); Schultz, John R., Burbank, CA 91501 (US); Powell, David G., South Pasadena, CA 91030 (US); Spell, Judith D., Nashville, Tennessee 37221-1907 (US)
(74) Representative: Rees, David Christopher

(56) References cited:
- EP-A- 0 048 423
- EP-A- 0 183 351
- US-A- 4 985 015

## Description

This invention relates generally to an apparatus for delivering medication to a patient including an implantable infusion pump comprising for quickly and easily detecting a condition adversely affecting medication delivery in the implantable medication infusion pump, and more particularly to an apparatus for reliably detecting occurrences including an occluded catheter, and the presence of air in the pumping mechanism.

Medication infusion pumps are generally known in the art for use in delivering a selected medication to a patient in a scheduled or preprogrammed manner. In recent years, such medication infusion pumps have been developed in compact form adapted for implantation into the body of a patient. They are used to deliver a specific medication such as insulin to the patient in discrete (but essentially continuous) doses over an extended time period.

An implantable medication infusion pump of this general type typically includes an internal medication reservoir for receiving and storing a supply of the selected medication in liquid form. Other components incorporated in the device include a power source (typically a battery), a miniature pumping mechanism, and associated electronic programmed control means for delivering the medication to the patient according to a prescribed schedule. For one illustrative example of an implanted or implantable medication infusion pump of this general type, see U.S. Patent No. 4,573,994, to Fischell.

Implantable medication infusion pumps are normally equipped with an inlet port through which fluid medication can be supplied to permit periodic refilling of the pump reservoir. This inlet port is typically positioned and shaped for receiving a transcutaneous needle through which the fluid medication is supplied from outside the patient's body. Accordingly, the pump reservoir can be filled or refilled without requiring surgical, removal from the patient's body, and further without requiring any other significant surgical procedure.

The output port of an implantable medication infusion pump is typically connected to the proximal end of a catheter. The distal end of the catheter is located in the abdominal cavity, where it may either be free-floating or implanted in the tissue of the omentum. If the distal end is to be free-floating, which is currently seen as the better technique, an intermediate portion of the catheter having a 90 degree bend therein is anchored to the peritoneal wall. If the distal end is to be placed into a fold in the omentum tissue, the tissue is sutured around the distal end of the catheter. The medication is thus delivered by the implantable medication infusion pump through the catheter to the body of patient.

While implantable, refillable medication infusion pumps constitute a major step forward in reliable and convenient administration of certain medications, there are several conditions which may be encountered which are difficult to detect, and which may impede the efficacious delivery of medication to the patient. The most significant of these problems is the problem of a partially or completely blocked catheter. This is typically caused by one of two blocking mechanisms: first, by tissue growth encapsulating substantially the entire distal end of the catheter to form a "sock;" or secondly, by deposits aggregating inside the lumen of the catheter to block or occlude the lumen of the catheter. Either of these conditions may result in the flow of medication from the catheter being partially or fully obstructed.

A catheter may become partially encapsulated or occluded, which will act to raise the pressure in the catheter when medication is being delivered. More infrequently, a catheter may become virtually fully encapsulated or occluded, essentially preventing the medication from flowing from the distal end of the catheter. Such a condition will defeat the intended purpose of the implanted system by substantially preventing it from delivering medication.

In delivering medications such as insulin, periodic relatively larger doses (boluses) are periodically supplied as needed (such as immediately before meals) in addition to an essentially continuous supply of the medication at a low rate (basal rate). With a partially obstructed catheter, the delivery of medication at the basal rate may not be a problem, while the delivery of a larger bolus may cause pressure to build to a point where delivery is significantly impeded. It will be recognized by those skilled in the art that nondelivery of medication due to a catheter obstruction thus represents a significant problem.

Another problem encountered by implantable medication infusion pumps is the presence of a gas bubble in the pumping mechanism. This is a particularly serious problem in medication infusion pumps which are incapable of pumping gas. Still another problem is that of pump failure, in which the pumping mechanism simply stops working for whatever reason. These problems, like the problem of catheter obstruction, may result in vital medication not being delivered to a patient.

At present, the lack of medication being delivered to a patient is generally detected by physiological measures. Such measures, for example, may include blood glucose analysis (for insulin), surgical procedures such as laparoscopy, or the use of an invasive pressure measuring technique such as a side port. These measures are all effective at diagnosis only significantly after the problem has been encountered for some time. In the case of a partially obstructed catheter, these techniques may not be successful in making a correct diagnosis.

A promising development has been the use of an acoustic sensor built into the pumping mechanism of the implantable medication infusion pump, which is taught in U.S. Patent. No. 4,985,015 (the '015 patent), used as a basis for the preamble of claim 1.

This device uses a piezo element located in the pumping mechanism to sense noise, with the noise sensed (or not sensed) providing information about medication infusion pump operation. The '015 patent teaches that the amplitude and timing of the acoustic signal present information useful to diagnosis of various pumping conditions.

Specifically, with regard to the timing of a "normal" signal the '015 patent teaches that an empty reservoir is indicated when the signal is greatly premature, and that air bubbles in the pumped fluid are indicated when the signal is slightly premature. With regard to the amplitude of the acoustic signal, the '015 patent teaches an amplitude or threshold discriminator which produces a signal when the medication infusion pump is operating properly, and no signal when the catheter is occluded. In the event of a catheter occlusion beginning to occur, there will be a signal when pumping is started, which signal will disappear when pumping at a high rate (read pumping a bolus).

Thus, the '015 patent teaches the use of a threshold detected acoustic signal, the timing and presence of which provides operational information on the system. This is certainly a significant improvement in the detection of the problems mentioned above. However, the system of the '015 patent has certain disadvantages representing opportunities for improvement in the art.

The biggest problem with the '015 patent is that it requires the provision of the acoustic detection and diagnosis system in the implantable medication infusion pump. Thus, the system of the '015 patent is useless on all currently implanted medication infusion pumps not having the system installed in the medication infusion pump. Given the high degree of governmental regulation involved in the medical device area, it will likely be years before an implantable medication infusion pump embodying the device of the '015 patent will be available.

It is accordingly an object of the present invention that it provides an improved apparatus for detecting the problems mentioned above in the implanted medication infusion pump. The apparatus for detecting problems in the implanted medication infusion pump must be capable of discriminating between problems such as an encapsulated or occluded catheter and a nonoperational pumping mechanism.

The apparatus must be highly accurate in detecting problems in the implanted system allowing a correct diagnosis of problems to be made with a high degree of accuracy while avoiding entirely an incorrect diagnosis which could result in the removal of a properly functioning system.

The apparatus for detecting problems in an implanted medication infusion pump should also be economical, and relatively simple to perform. Interference with spurious signals must be avoided. In addition, a means should be provided to enable the analysis to be tailored to individual patients, to better follow the conditions of these patients. It is also an objective that all of the aforesaid advantages and objectives be achieved without incurring any substantial relative disadvantage.

It has been established by Obermann at al. in the '015 patent that by using an acoustic sensor in an implanted medication infusion pump, an acoustic signal will be provided when the pumping mechanism, typically a piston-type pump, operates. This signal in the '015 patent is phase and level detected, and has been found to be indicative, to the extent of the limitations discussed above, of the state of the implanted system. The present invention improves substantially on the basic concept by performing a more complete analysis on the level of the signal, rejecting the essentially digital level detection of the '015 patent for a more complete analysis which will yield more complete information as to the state of the system.

The invention is defined in the independent claim appended hereto. Preferred features are set out in the dependent claims.

The signal obtained for a system with an encapsulated or occluded catheter is significantly smaller than the signal for a "normal" system. In addition, it has been determined that the signal obtained for a system with air contained in the pumping path is substantially larger than the signal for a "normal" system. These fundamental facts are true both in the time domain and in the frequency domain. Thus, by storing appropriate baseline values in memory within the implanted medication infusion pump, and later comparing these stored baseline values with corresponding values measured later, the condition of the implanted medication infusion system may be evaluated.

It has been discovered that by using an electronic stethoscope placed on the skin over the implanted medication infusion pump, an acoustic signal may be obtained when the pumping mechanism, typically a piston-type pump, operates. This signal has been found to be highly indicative of the state of the implanted system. In the preferred embodiment, the signal obtained from the electronic stethoscope is first filtered to remove extraneous body noises. It may advantageously be recorded, preferably on a digital recorder, for further analysis.

The signal may then be analyzed, both in the time domain and in the frequency domain. It has been determined that the signal obtained for a system with an encapsulated or occluded catheter is significantly smaller than the signal for a "normal" system. In addition, it has been determined that the signal obtained for a system with air contained in the pumping path is substantially larger than the signal for a "normal" system. These fundamental facts are true both in the time domain and in the frequency domain.

In the preferred embodiment, the value looked for in the time domain is the peak voltage, and in the frequency domain is the peak sound level at the peak frequency. RMS values or other average values may also be utilized. Alternately, the sound level at a particular frequency may be looked at in the frequency domain. In addition, the integral of the sound level in the frequency domain or the integral of the magnitude of the voltage in the time domain (or their averages, which are proportional to the integrals) may be used. All of these indicia have been demonstrated to provide a clear indication of the state of the system.

Partial encapsulations or occlusions may also be diagnosed by monitoring successive signals with the system delivering medication with a rapid sequence of pump strokes. Over a sequence of rapid, successive pump strokes a falling acoustic level is indicative of a partial encapsulation or occlusion.

In the preferred embodiment, it is preferred that a baseline reading of each patient be obtained to provide a means for a comparison if a problem should arise. The baseline readings may vary widely from patient to patient, due to different physiological elements such as weight of the patient and implantation location. A comparison of readings from a malfunctioning system with the baseline readings provides a dramatic indication of the efficacy of the comparison in diagnosing pump problems (or the lack thereof).

The preferred embodiment also includes an interconnection between the monitoring system of the present invention and the control circuitry of the implanted medication infusion pump. In the event of a problem, an alarm can be provided through known means such as low level "tickle" electrical stimulation of the patient, a buzzer, and/or through telemetry to the patient and physician communicators. If desired, the system may also be shut down.

It may therefore be seen that the present invention teaches an external apparatus for detecting the problems mentioned above in an implanted medication infusion pump. The apparatus is highly accurate in detecting problems in the implanted system, and allows a correct diagnosis of problems to be made with a high degree of accuracy while avoiding entirely an incorrect diagnosis which could result in the removal of a properly functioning system.

The apparatus of the present invention is capable of discriminating between problems such as an encapsulated or occluded catheter and a nonoperative pumping mechanism. It is also capable of determining the presence of air in the system.

The apparatus for detecting problems in an implanted medication infusion pump is relatively inexpensive, and its method of use is relatively simple to perform. Interference with spurious signals is avoided by filtering, and the entire operation is noninvasive. In addition, by obtaining baseline data the analysis is specifically tailored to individual patients, to thereby better follow the conditions of these patients.

The invention may be carried into practice in various ways and some embodiment will now be described by way of example with reference to the accompanying drawings, in which:-
Fig. 1 is a somewhat schematic depiction of the transducer of the present invention located over an implanted medication infusion pump in the abdomen of a patient in position for monitoring the acoustic signals generated by the pump, and also showing the control module used to process the signal from the transducer, the digital tape recorder used to record the processed signal, and the oscilloscope used to display the processed signal for comparison;
Fig. 2 is a block diagram illustrating in schematic fashion the system of the present invention illustrated in Fig. 1;
Fig. 3 is a waveform illustrating the filtered, amplified signal from the acoustic sensor in the time domain for a normally operating implanted medication infusion system;
Fig. 4 is a waveform illustrating the filtered, amplified signal from the acoustic sensor in the time domain for an implanted medication infusion system having a completely encapsulated or occluded catheter;
Fig. 5 is a waveform illustrating the filtered, amplified signal from the acoustic sensor in the frequency domain for a normally operating implanted medication infusion system;
Fig. 6 is a waveform illustrating the filtered, amplified signal from the acoustic sensor in the frequency domain for an implanted medication infusion system having a completely encapsulated or occluded catheter;
Fig. 7 illustrates for comparison a waveform illustrating the filtered, amplified signal from the acoustic sensor in the frequency domain for a normally operating implanted medication infusion system, and a waveform illustrating the filtered, amplified signal from the acoustic sensor in the frequency domain for an implanted medication infusion system having air in the fluid line;
Fig. 8 is a waveform illustrating the relative peak level of the filtered, amplified signal from the acoustic sensor for an implanted medication infusion system having a partially encapsulated or occluded catheter plotted against pulses of fluid which the system is continuously delivering; and
Fig. 9 is a waveform illustrating the relative RMS spectrum level of the filtered, amplified signal from the acoustic sensor for an implanted medication infusions system having a partially encapsulated or occluded catheter plotted against pulses of fluid which the system is continuously delivering.
Fig. 10 is a cutaway view of an implantable medication infusion pump; and
Fig. 11 is a block diagram illustrating in schematic fashion the system illustrated in Fig. 10, with particular attention to the components of the acoustic monitoring system.

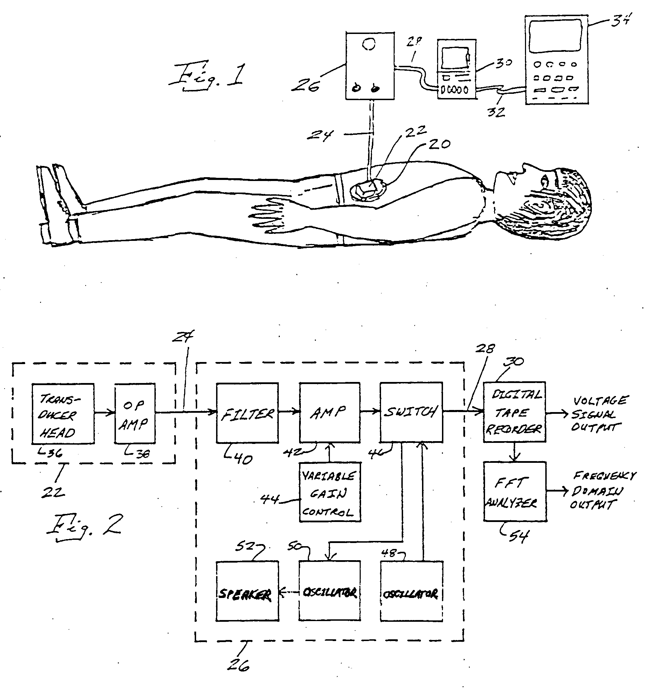

Referring first to Fig. 1, an individual is illustrated who has an implanted medication infusion pump 20 implanted in his abdomen below a layer of skin and tissue. It has been discovered that by placing a electronic stethoscope head 22 over the skin overlying the implanted medication infusion pump 20, the acoustic signals generated by the implanted medication infusion pump 20 may be monitored. The electronic stethoscope head 22 is centered on the skin of the individual over the implanted medication infusion pump 20.

Thus, as the pump in the implanted medication infusion pump 20 (which is typically a piston type pump) operates, the electronic stethoscope head 22 will generate electrical signals characteristic of the acoustic signal picked up from the implanted medication infusion pump 20. This electrical signal will be transmitted through wires 24 to a control module 26, where the electrical signal from the electronic stethoscope head 22 is processed. The processed electrical signal may then be transmitted via wires 28 to a digital tape recorder 30 for storage of the processed signals from the electronic stethoscope head 22.

The stored processed signals from the electronic stethoscope head 22 may be played back by the digital tape recorder 30 and transmitted via wires 32 to an oscilloscope 34. The oscilloscope 34 will thus display the stored processed. signals from the electronic stethoscope head 22. Comparison of the stored processed signals from the electronic stethoscope head 22 is made with other data to analyze the recorded information.

In this manner, the acoustic signals generated by the implanted medication infusion pump 20 are recorded for later analysis and comparison. Note that instead of using the digital tape recorder 30, the processed signals from the electronic stethoscope head 22 could be immediately analyzed and displayed on the oscilloscope 34. The digital tape recorder 30 is used to facilitate storage of the processed signals from the electronic stethoscope head 22 so that they may be analyzed in more than one way, if desired, and at more than one time.

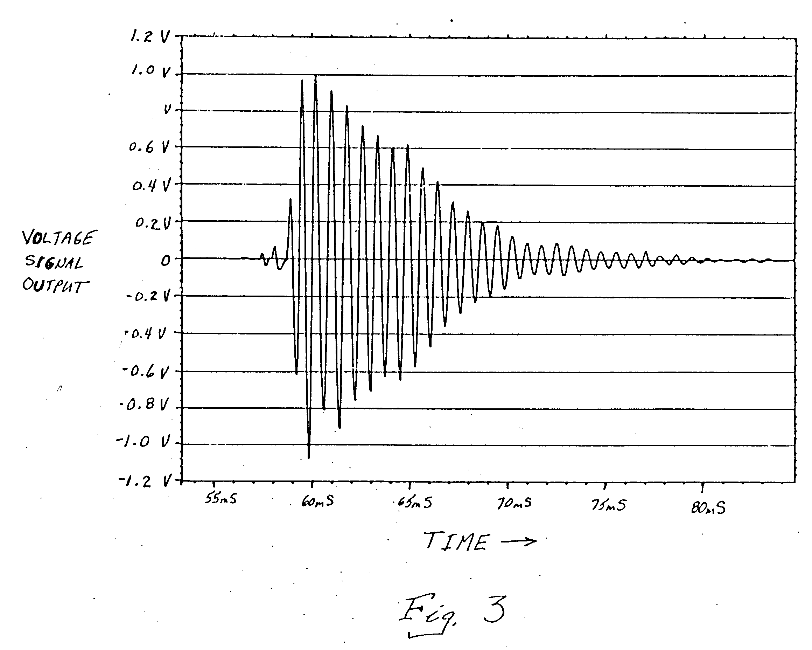

Referring now to Fig. 2, the apparatus of Fig. 1 is shown in block diagram format, with the oscilloscope 34 omitted, and with an additional component which may be used to analyze the signals in the frequency domain. It will be understood that the oscilloscope 34 may be used with the components of Fig. 2 to display the resulting outputs of the system of Fig. 2.

Referring first to the electronic stethoscope head 22, in the preferred embodiment a commercial electronic stethoscope transducer head 36 has been modified and used. For example, the transducer head from an electronic stethoscope available from Andries Tek, Inc. of Austin, Texas may be modified to be suitable. This particular electronic stethoscope transducer head 36 has an electrical output which may be utilized.

The Andries Tek Model 1200 electronic stethoscope transducer head 36 has a 500 Hz low pass filter characteristic. However, the sounds which a medical stethoscope is designed to pick up are body sounds, which in the application considered herein are merely noises. Accordingly, either the Andries Tek electronic stethoscope transducer head 36, must be modified or the control module 26 must include appropriate filter circuitry to exclude undesirable body noises and to maximize the detection of acoustic signals from the implanted medication infusion pump 20. In the preferred embodiment both of these solutions are used together.

It has been experimentally determined that the frequencies of relevant signals obtainable from the implanted medication infusion pump 20 are between 1 KHz and 5 KHz. Accordingly, in the preferred embodiment, the Andries Tek electronic stethoscope transducer head 36 is modified to have 5 KHz low pass characteristics. In addition, in the preferred embodiment an op amp used in the Andries Tek electronic stethoscope is replaced with an op amp 38 which has lower noise (and low power) characteristics, such as an LM660 op amp. The op amp 38 is thus used in the electronic stethoscope head 22 to amplify the signals detected by the Andries Tek electronic stethoscope transducer head 36.

The electrical signal from the electronic stethoscope head 22 is supplied to the control module 26, which includes a high pass filter 40 and an amplifier 42 having a variable gain control 44. The electrical signal from the electronic stethoscope head 22 is thus passed through the high pass filter 40, which eliminates the portion of the signal characteristic of body noises. In the preferred embodiment, the high pass filter 40 is a 1 KHz high pass filter.

The amplifier 42 is then adjusted by the variable gain control 44 to produce an appropriate output level which can be displayed on the oscilloscope 34 (Fig. 1). In the preferred embodiment, the amplifier 42 is capable of producing gains of 0, 10, 20, and 30 dB. The output of the amplifier 42 is the filtered, amplified signals detected by the electronic stethoscope head 22.

In the preferred embodiment, the control module 26 also includes means for calibrating the system. A three position switch 46 is used to control the operation of the control module 26. In a first position, the three position switch 46 passes the filtered, amplified signals detected by the electronic stethoscope head 22 as the output from the control module 26. In a second position, the three position switch 46 provides as the output of the control module 26 an electrical calibration signal, which in the preferred embodiment is a 1600 HZ, 100 mV RMS signal. This signal is supplied by an oscillator 48 to enable an electrical calibration of the system to be made.

In a third position, the three position switch 46 provides the filtered, amplified signals detected by the electronic stethoscope head 22 as the output from the control module 26, but activates an oscillator 50 which produces an acoustic tone from a speaker 52, which tone in the preferred embodiment is 2 Pascal pressure at 1600 Hz. The electronic stethoscope head 22 is placed adjacent the speaker 52 to enable an acoustic calibration of the system to be made so that a 100 mV RMS output is produced.

The filtered, amplified signals detected by the electronic stethoscope head 22 are thus supplied to the digital tape recorder 30. The digital tape recorder 30 may be, for example, a portable Sony digital audio tape recorder, Model TCD-D3. Other recording devices could also be used, but the digital tape recorder 30 is preferred because of its high accuracy in reproducing the recorded filtered, amplified signals detected by the electronic stethoscope head 22.

In operation, the electronic stethoscope head 22 will produce an acoustic signal each time the pumping mechanism in the implanted medication infusion pump 20 is operated. The signal will be appropriately filtered and amplified, and either supplied to the digital tape recorder 30 for storage and later playback, or, alternately, immediately displayed on the oscilloscope 34 (Fig. 1).

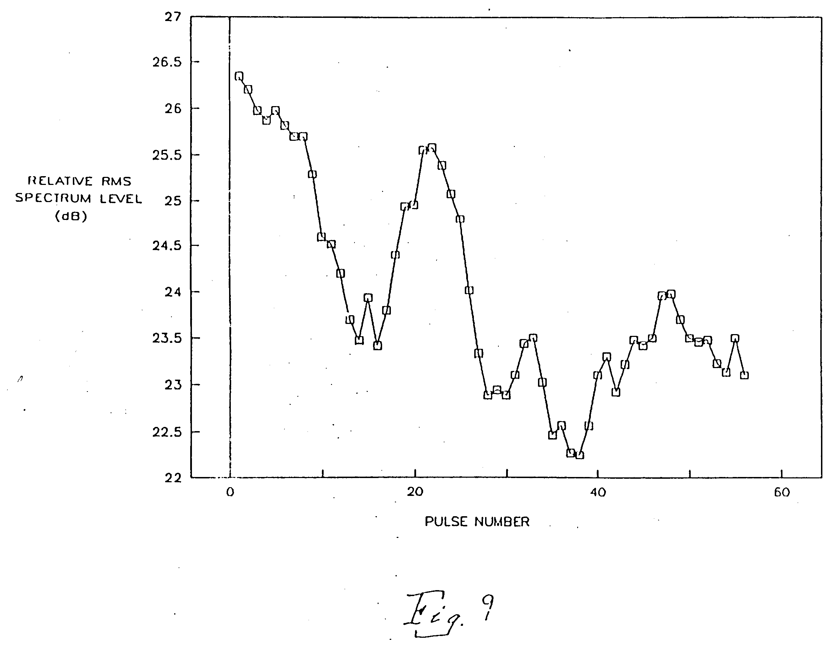

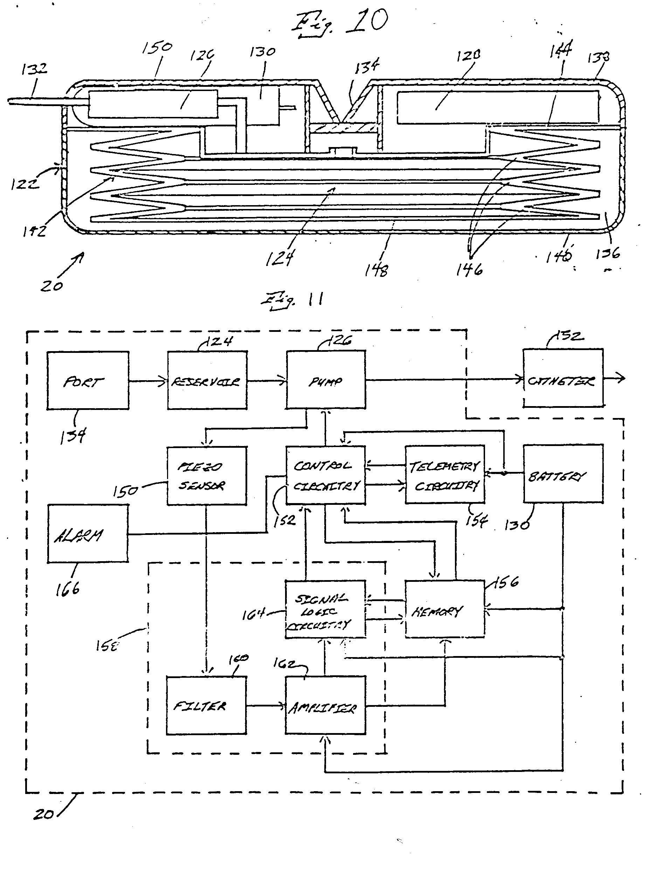

As background information an illustrative medication infusion pump 20, as shown in Fig. 10, and which is not in accordance with the present invention, comprises a small and substantially self-contained unit designed for direct implantation into the body of a patient. The medication infusion pump 20 comprises a hermetically sealed pump housing 122 constructed from a biocompatible material such as titanium or titanium alloy. The bottom portion of the hermetically sealed pump housing 122 defines the internal medication reservoir 124 for receiving and storing the supply of the selected medication in liquid form, such as insulin for a diabetic patient.

The hermetically sealed pump housing 122 further encases a miniature dispensing pump 126 and generally depicted associated electronic control circuitry 128 in combination with a battery 130 for periodically operating the pump 126 to deliver medication doses from the reservoir 124 to the patient via an appropriate catheter 132 or the like. The general control circuitry includes circuitry which is suitably preprogrammed to deliver the medication in accordance with individual patient need. An inlet or refill port 134 on the hermetically sealed pump housing 122 is adapted to receive a hypodermic needle (not shown) to permit percutaneous refilling of the reservoir 124 without requiring surgical access to the medication infusion pump 20. For a more detailed description of the overall construction and operation of implantable infusion pumps of this general type, see U.S. Patent Nos. 4,373,527 and 4,573,994.

As is known in the art, the medication infusion pump 20 includes the variable volume pressure reservoir 136 mounted within the hermetically sealed pump housing 122 with at least one movable wall of the pressure reservoir 136 being shared with and thereby defining at least a portion of the reservoir 124. More particularly, the pressure reservoir 136 contains a selected pressure fluid adapted to vary the volumetric size of the reservoir 124 in accordance with the quantity of medication therein to maintain the medication under substantially constant pressure conditions.

The preferred pressure fluid is a fluorocarbon which has a substantially linear pressure characteristic as it changes from liquid to vapor state and vice versa at normal human body temperature and at a normal range of altitudes. A preferred pressure fluid is Freon 113 (a Trade Mark) which assumes a liquid-vapor state at normal body temperature and at altitudinal variations up to about 8,500 feet (2.591m) above sea level to exert a slightly negative and substantially constant pressure of approximately -1.0 to -4.5 psi (-0.7 to 3.2 kg/mm²) on the reservoir 124. A positive pressure reservoir could instead be used.

The illustrative drawings show the assembled hermetically sealed pump housing 122 in the form of interfitting upper and lower housing members 138 and 140 of generally circular and shell-shaped configuration. In general terms, the upper housing member 138 has the pump 126 and the general control circuitry with the associated battery 130 installed therein. By contrast, the lower housing member 140 has a bellows unit 142 installed therein. The bellows unit 142 is shown with an upper ring 144 of generally annular shape having an outer periphery secured in sealed relation to an inboard side of a circular wall on the lower housing member 140, and an inner periphery joined to a plurality of downwardly extending bellows corrugations 146.

The bellows corrugations 146 of the bellows unit 142 are joined in turn to a circular lower plate 148. This structure defines a pump reservoir subassembly with the volumetric space disposed radially within the bellows unit 142 defining the reservoir 124, and the volumetric space located radially outside and axially below the bellows unit 142 defining the pressure reservoir 136. When the medication infusion pump 20 is finally assembled, it will be understood that the upper housing member 138 fits over the lower housing member 140 to define and close the upper region of the reservoir 124 in operative relation with the pump 126.

An acoustic transducer 150, is visible in Fig. 10 which is mounted between the pump 126 and the upper housing member 138. The acoustic transducer 150 is a piezoelectric element, such as a segment of Kynar (a Trade Mark) which is mounted on the pump 126. When the pump 126 pulses, the piezo sensor 150, as it will hereafter be called, will generate an electrical signal indicative of the acoustic signal generated by the pulsing of the pump 126.

Referring for background information next to Fig. 11, which is also not in accordance with the present invention, a block diagram illustrating how the components of the apparatus are configured is presented. The fluid path from the port 134 to the reservoir 124, from the reservoir 134 to the pump 126, and from the pump 126 to the catheter 132 are illustrated. The pump 126 mechanically drives the piezo sensor 150 to produce an electrical output.

The general control circuitry 128 of Fig. 10 is illustrated in Fig. 11 as four elements: control circuitry 152 for operations of the pump as is generally known in the art; telemetry circuitry 154, also known in the art; memory 156, also known in the art; and processing circuitry 158 for processing the signal from the piezo sensor 150. The telemetry circuitry 154 is connected to send data to and to receive data from the control circuitry 152. The control circuitry 152 is connected to drive the pump 126. The electrical output from the piezo sensor 150 is supplied to the processing circuitry 158, which supplies an output signal to the control circuitry 152.

The processing circuitry 158 includes a filter 160, which filters the electrical signal from the piezo sensor 150. The filtered signal from the piezo sensor 150 is supplied to an amplifier 162, which supplied the amplified filtered signal from the piezo sensor 150 to signal logic circuitry 164 and to the memory 156. The output from the signal logic circuitry 164 is the output signal of the processing circuitry 158, which is supplied to the control circuitry 152.

The memory 156 is connected to receive data from and to send data to both the signal logic circuitry 164 and the control circuitry 152. The battery is connected to power the entire system, including the control circuitry 152, the telemetry circuitry 154, the memory 156, the amplifier 162, and the signal logic circuitry 164. An alarm mechanism 166 may be included to provide an indication of a system malfunction.

In operation, the piezo sensor 150 produces a signal each time the pump 126 is operated. The pump 126, which is typically a piston-type pump, will produce an acoustic signal whenever it is operated. In response, the piezo sensor produces an output signal each time the pump 126 is pulsed.

The filter 160 preferably includes a high pass filter which cuts off the portion of the signal below 1000 Hz. This eliminates body noise, which has been found to be necessary for proper signal discrimination. The preferred embodiment is a bandbass filter passing signals between 1000 Hz and 5000 Hz.

The signal from the piezo sensor 150 is thus filtered by the filter 160, and is then amplified by the amplifier 162. At this point, a voltage waveform representative of the acoustic signal generated by the pump 126 exists, which may be stored in the memory 156. This signal is also supplied to the signal logic circuitry 164, where it is analyzed.

At this point it is necessary to note that a number of different analyses may be performed. The basic analysis is a comparison between the baseline voltage signal and the current voltage signal. It has been determined that a signal from a system which is "normal" has a first value in the time domain. A system with an encapsulated or occluded catheter has a value in the time domain which is at a substantially lower level than the "normal" signal. A system with air in the fluid line has a value in the time domain which is at a higher level than the "normal" signal.

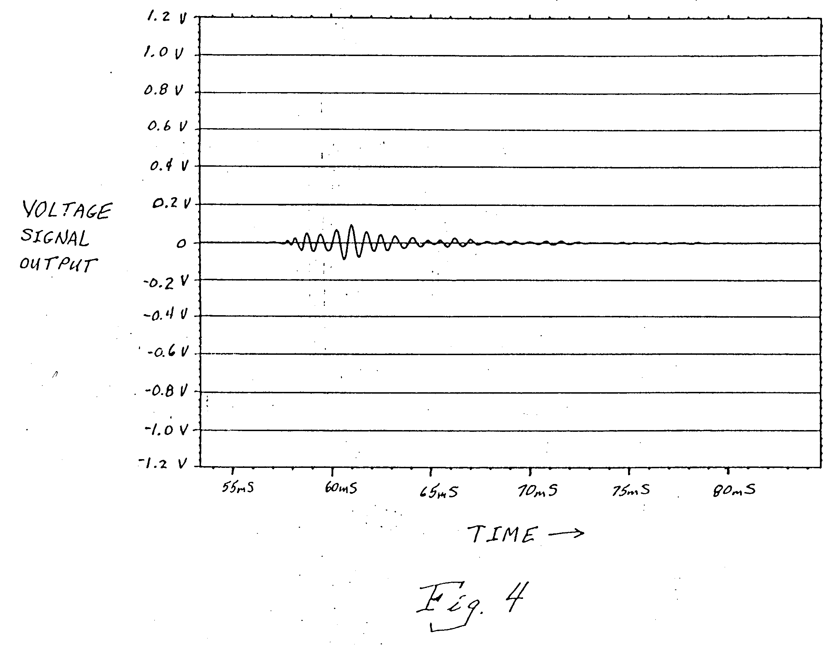

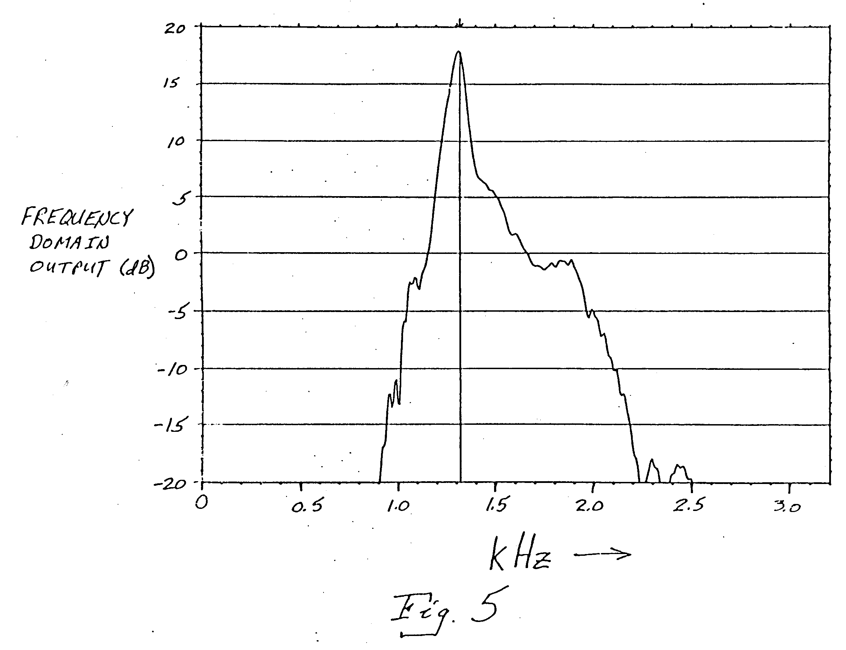

The preferred embodiment uses the peak voltage signal as the sensed parameter. Thus, a system with an encapsulated or occluded catheter has a peak voltage in the time domain which is substantially lower than the peak voltage of the "normal" signal in the time domain. This is apparent in comparing Figs. 3 and 4. Fig. 3 shows the time domain waveform for a "normal" system, while Fig. 4 shows the time domain waveform for an encapsulated or occluded catheter. The signals, including the peak signal, are over ten times higher for a "normal" system than for a system with an encapsulated or occluded catheter. As a cautious minimum, when the current signal is one-fifth of the baseline signal or less, an encapsulated or occluded catheter is indicated.

In addition, a system with air in the fluid line has a peak voltage in the time domain which is higher than the peak voltage of the "normal" signal in the time domain. The peak signal in the time domain for a system with air in the fluid line is two to three times greater than the peak voltage in the time domain for a "normal" system. As a cautious minimum, when the current signal is twice the baseline signal or more, air in the fluid line is indicated.

In the time domain, the average, the RMS average, or the integral of the voltage signal could also be used instead.

In addition, the frequency content of the signal could be used instead. If the frequency domain of the signals are calculated (using an FFT analyzer 54 on the output from the digital tape recorder 30, or, directly, on the output from the control module 26), the peak value of the frequency domain signals has also been found to be indicative of the condition of the system.

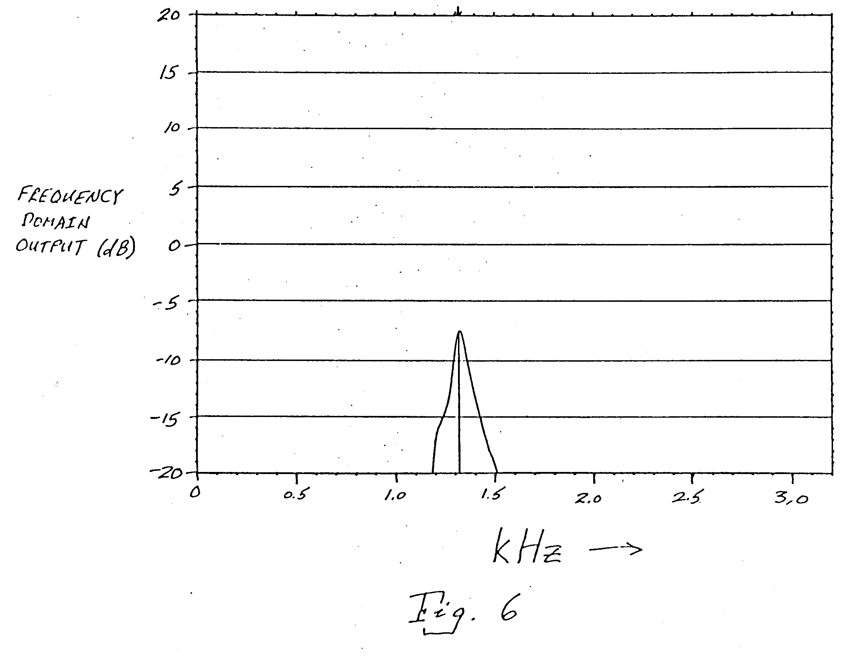

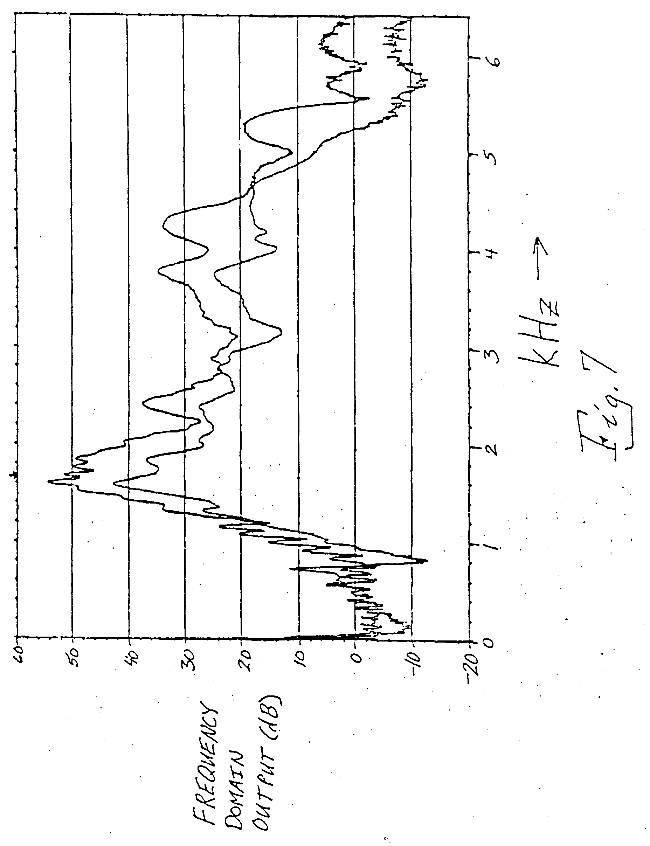

Thus, a system with an encapsulated or occluded catheter has a peak value in the frequency domain which is substantially lower than the peak value of the "normal" signal in the frequency domain. This is apparent in comparing Figs. 5 and 6. Fig. 5 shows the frequency domain waveform for a "normal" system, while Fig. 6 shows the frequency domain waveform for an encapsulated or occluded catheter. The signals, including the peak signal, are over 20 dB higher for a "normal" system than for a system with an encapsulated or occluded catheter.

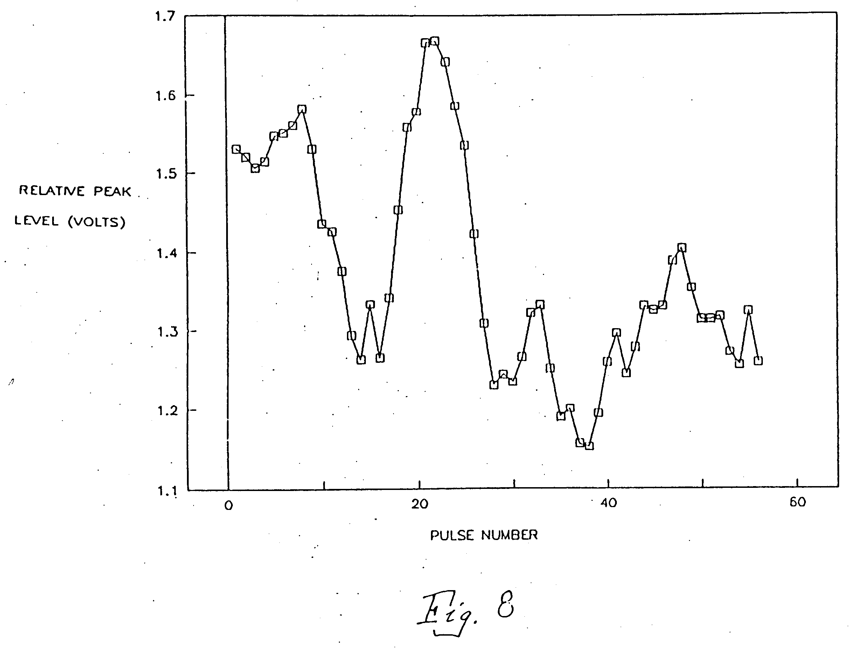

In addition, a system with air in the fluid line has a peak value in the frequency domain which is 5 to 10 dB higher than the peak value of the "normal" signal in the frequency domain. This is illustrated in Fig. 7. The upper waveform in Fig. 7 is for a system with air in the fluid line, and the lower waveform is for a "normal" system. It is apparent that the peak signal in the time domain for a system with air in the fluid line is 2 to 3 times greater than the peak voltage in the time domain for a "normal" system.

In the frequency domain, the value at a particular frequency could also be used instead of the peak value. Here, that frequency would be approximately 1320 Hz; typically, it is between 1200 Hz and 1700 Hz. In addition, the average, the RMS average, or the integral of the signal could also be used instead.

A partially encapsulated or occluded catheter may also be diagnosed, either in the time domain as shown in Fig. 8, or in the frequency domain as shown in Fig. 9. By plotting the peak time domain voltage versus pulses of the pump 26 (Fig. 8), or relative RMS spectrum level versus pulses of the pump 26 (Fig. 9), patterns emerge. There will be a downward slope of the plot in either case, indicating an encapsulation or occlusion, until the encapsulation or occlusion opens up, at which time the plot will slope upwards until pressure builds up again. Thus, a single or repeated downward slope while pumping during a bolus indicates a partial encapsulation or occlusion. As a cautious minimum, when repeated downward slope patterns are observed during repetitive, closely spaced pumping cycles, a partially encapsulated or occluded catheter is indicated.

In operation, when the implantable medication infusion pump 20 is first implanted, the system will build up baseline values indicative of proper operation. Later, as the device operates and as current values are generated, these new values will be compared with the stored baseline values. A conclusion may thus be made as to the state of the system.

A current signal which is substantially lower than the baseline signal is indicative of an encapsulated or occluded catheter. A current signal which is significantly higher than the baseline signal is indicative of air in the fluid line. A current signal for a sequence of pulses which decrease in amplitude (either in a single sequence or in repeated sequences with brief returns to higher values) is indicative of a partially encapsulated or occluded catheter.

Specifically, when the current signal is one-fifth of the baseline signal or less, an encapsulated or occluded catheter is indicated. When the current signal is twice the baseline signal or more, air in the fluid line is indicated. When repeated downward slope patterns are observed during repetitive, closely spaced pumping cycles, a partially encapsulated or occluded catheter is indicated.

If desired, the memory 156 may be used to periodically store values of the signals at different times. This information may be telemetered out later to review whether there is a trend indicating that an encapsulation or occlusion in the catheter 132 is about to occur.

If the system is operating improperly, an alarm may be given through conventional means, designated generally in Fig. 11 as the alarm mechanism 166. Examples of such means include built-in alarms (included within the control circuitry 152) such as a low level "tickle" electrical stimulation of the patient or a buzzer, or through telemetry to patient and physician communicators (not shown). If desired, the system may also be shut down by the control circuitry 152.

It may therefore be appreciated from the above detailed description of the preferred embodiment of the present invention that it teaches an external apparatus for detecting the problems mentioned above in an implanted medication infusion pump. The apparatus is highly accurate in detecting problems in the implanted system, and allows a correct diagnosis of problems to be made with a high degree of accuracy while avoiding entirely an incorrect diagnosis which could result in the removal of a properly functioning system.

The apparatus of the present invention is capable of discriminating between problems such as an encapsulated or occluded catheter and a nonoperative pumping mechanism. It is also capable of determining the presence of air in the system.

The apparatus for detecting problems in an implanted medication infusion pump is relatively inexpensive, and is relatively simple to perform. Interference with spurious signals is avoided by filtering, and the entire operation is noninvasive. In addition, by obtaining baseline data the analysis is specifically tailored to individual patients, to thereby better follow the conditions of these patients. Finally, all of the aforesaid advantages and objectives are achieved without incurring any substantial relative disadvantage.

## Claims

1. An apparatus for delivering medication to a patient including an implantable medication infusion pump comprising a reservoir for containing a fluid medication and means (20) for transferring the fluid medication from the reservoir to a location outside the implantable medication infusion pump; the apparatus further incorporating means (22) for generating electrical signals proportional to acoustic signals generated by the implanted medication infusion pump; means (26) for processing the electrical signals to produce processed electrical signals; means (34) for displaying the processed electrical signals to determine characteristics related to their relative amplitude which are indicative of the operational condition of the implanted medication infusion pump; and logic means (54, 164) arranged to utilise the characteristics related to the relative amplitude of the processed electrical signals to determine the operational condition of the implanted medication infusion pump, the logic means including means for providing an informational signal if the logic means determine the existence of a system malfunction; characterised in that the means (22) for generating electrical signals are means for non-invasively generating electrical signals, the logic means (54, 164) providing a first informational signal if the processed electrical signals are greater than or equal to a first predetermined value, the logic means providing a second informational signal if the processed electrical signals are smaller than or equal to a second predetermined value, the second predetermined value being smaller than the said first predetermined value.

2. An apparatus as claimed in Claim 1, characterised in that the processing means (26) comprise filtering means (40) which filter the electrical signals to produce filtered electrical signals; and the logic means (54) are arranged for comparing the characteristics related to the relative amplitude of the filtered electrical signals to characteristics of baseline electrical signals related to their relative amplitude to determine the operational condition of the implanted medication infusion pump.

3. An apparatus as claimed in Claim 1, characterised in that the processing means (26) comprise filter means (40) which filter the electrical signals to produce filtered electrical signals; and the displaying (34) or logic means (54) are arranged to use characteristics related to the slope of their relative amplitude of the filtered signals during repetitive, closely spaced pumping cycles to determine the operational condition of the implanted medication infusion pump.

4. An apparatus as claimed in any preceding Claim, characterised in that the transferring means (20) comprises: a piston-type pump.

5. An apparatus as claimed in any preceding Claim, characterised in that the generating means comprises: an electronic stethoscope head (22) mounted to pick up acoustic signals generated by the transferring means.

6. An apparatus as claimed in Claim 2 or 3, characterised in that the filtering means (40) comprises a high pass filter to eliminate substantially body noise in the electrical signals, the high pass filter preferably being arranged to remove signals below approximately 1 KHz.

7. An apparatus as claimed in Claim 6, characterised in that the filter means (40) additionally comprises a low pass filter to remove signals above approximately 5 KHz from the electrical signals.

8. An apparatus as claimed in any preceding Claim, characterised in that the processing means (26) additionally comprises: an amplifier (42) to amplify said electrical signals.

9. An apparatus as claimed in Claim 1, characterised in that the logic means (54, 164) comprises: means for determining a characteristic related to the relative amplitude of the processed electrical signals; and means for comparing the characteristic related to the relative amplitude of the processed electrical signals to a characteristic of baseline electrical signals related to their relative amplitude to determine the operational condition of the implanted medication infusion pump, the first predetermined value being substantially larger than the characteristic of the baseline electrical signals related to their relative amplitude, the second predetermined value being substantially smaller than the characteristic of the baseline electrical signals related to their relative amplitude.

10. An apparatus as claimed in Claim 9, characterised in that the comparing means is arranged to indicate: an encapsulated or occluded catheter when the relative amplitude of the processed electrical signals is smaller than or equal to the second predetermined value.

11. An apparatus as claimed in Claim 10, characterised in that the comparing means is arranged to indicate an encapsulated or occluded catheter when the relative amplitude of the processed electrical signals is approximately one-fifth of the relative amplitude of the baseline electrical signals or less, the second predetermined value thus being approximately one-fifth of the amplitude of the baseline electrical signals.

12. An apparatus as claimed in Claim 11, wherein the comparing means is arranged to indicate an encapsulated or occluded catheter when the relative amplitude of the processed electrical signals is smaller than the relative amplitude of the baseline electrical signal or equal to the second predetermined value.

13. An apparatus as claimed in any of Claims 10 to 12, characterised in that the comparing means is arranged to indicate the presence of air in the fluid line when the relative amplitude of the processed electrical signals is larger than the first predetermined value.

14. An apparatus as claimed in Claim 13, characterised in that the comparing means is arranged to indicate that the presence of the processed electrical signals is approximately twice the relative amplitude of the baseline electrical signals or more, the first predetermined value thus being approximately twice the amplitude of the baseline electrical signals.

15. An apparatus as claimed in any of Claims 9 to 14, characterised in that the characteristics of the processed electrical signals and the baseline electrical signals related to their relative amplitudes comprise: the peak voltage in the time domain of each of the processed electrical signals and the baseline electrical signals; or the average voltage in the time domain of each of the processed electrical signals and the baseline electrical signals; or the RMS average voltage in the time domain of each of the processed electrical signals and the baseline electrical signals; or the integral in the time domain of each of the processed electrical signals and the baseline electrical signals; or the peak level in the frequency domain of each of the processed electrical signals and the baseline electrical signals; or the level at a particular frequency, for example in the frequency domain of each of the processed electrical signals and the baseline of electrical signals.

16. An apparatus as claimed in any of Claims 9 to 14, characterised in that the characteristics of the processed electrical signals and the baseline electrical signals related to their relative amplitudes comprise: the average level in the frequency domain of each of the processed electrical signals and said baseline electrical signals; or the RMS average level in the frequency domain of each of the processed electrical signals and the baseline electrical signals; or the integral in the frequency domain of each of the processed electrical signals and the baseline electrical signals.

17. An apparatus as claimed in Claim 3, characterised in that the logic means (54) indicates a partially encapsulated or occluded catheter when repeated downward slope patterns are present.

18. An apparatus as claimed in any preceding Claim, characterised in that the informational signal provided when a system malfunction is determined comprises a low level "tickle" electrical signal or an audible signal.

19. An apparatus as claimed in any preceding Claim, characterised by means for inhibiting the operation of the transferring means if the logic means determines the existence of a system malfunction.

20. An apparatus as claimed in Claim 3, characterised by the logic means (54) providing a first informational signal if the slope is greater than or equal to a first predetermined value, the logic means providing a second informational signal if the slope is smaller than or equal to a second predetermined value, the second predetermined value being smaller than the first predetermined value.

## Patentansprüche

1. Gerät zur Verabreichung von Medikamenten an einen Patienten, ausgestattet mit implantierbarer Medikamentinfusionspumpe, die einen Behälter zur Aufnahme flüssiger Medikamente und ein Hilfsmittel (20) zum Transportieren der flüssigen Medikamente vom Behälter der implantierbaren Medikamentinfusionspumpe nach außen umfaßt; Gerät enthält außerdem: ein Hilfsmittel (22) zur Erzeugung elektrischer Signale, die proportional zu den durch die implantierte Medikamentinfusionspumpe erzeugten akustischen Signalen sind; Hilfsmittel (26) zur Verarbeitung der elektrischen Signale, um verarbeitete elektrische Signale zu erzeugen; Hilfsmittel (34) zur Anzeige der verarbeiteten elektrischen Signale, um auf ihre relative Amplitude bezogene Kennziffern zu ermitteln, die auf den Betriebszustand der implantierten Medikamentinfusionspumpe schließen lassen: und Logikhilfsmittel (54, 164), zur Verwendung der auf die relative Amplitude der verarbeiteten elektrischen Signale bezogenen Kennziffern ausgelegt, um den Betriebszustand der implantierten Medikamentinfusionspumpe zu bestimmen, wobei die Logikhilfsmittel Hilfsmittel zur Bereitstellung eines Informationssignals umfassen, falls die Logikhilfsmittel eine Funktionsstörung des Systems feststellen; dadurch gekennzeichnet, daß die Hilfsmittel (22) zur Erzeugung elektrischer Signale Hilfsmittel zur nicht-invasiven Erzeugung elektrischer Signale sind, wobei die Logikhilfsmittel (54, 164) ein erstes Informationssignal bereitstellen, wenn die verarbeiteten elektrischen Signale größer gleich einem ersten vorbestimmten Wert sind, die Logikhilfsmittel ein zweites Informationssignal bereitstellen, wenn die verarbeiteten elektrischen Signale kleiner gleich einem zweiten vorbestimmten Wert sind, wobei der zweite vorbestimmte Wert kleiner als besagter erster vorbestimmter Wert ist.

2. Gerät nach Anspruch 1, dadurch gekennzeichnet, daß die Verarbeitungshilfsmittel (26) Filterhilfsmittel (40) enthalten, die die elektrischen Signale filtern, um gefilterte elektrische Signale zu erzeugen; ferner dadurch, daß die Logikhilfsmittel (54) zum Vergleich der auf die relative Amplitude der gefilterten elektrischen Signale bezogenen Kennziffern mit Kennziffern von elektrischen Impulsbodensignalen, die sich auf ihre relative Amplitude beziehen, ausgelegt sind, um den Betriebszustand der implantierten Medikamentinfusionspumpe zu bestimmen.

3. Gerät nach Anspruch 1, dadurch gekennzeichnet, daß die Verarbeitungshilfsmittel (26) Filterhilfsmittel (40) umfassen, die die elektrischen Signale filtern, um gefilterte elektrische Signale zu erzeugen; ferner dadurch, daß die Anzeigehilfsmittel (34) bzw. Logikhilfsmittel (54) ausgelegt sind, um auf die Steilheit der relativen Amplitude der gefilterten Signale bezogene Kennziffern während wiederholter, in kurzen Abständen stattfindender Pumpzyklen zu verwenden, um den Betriebszustand der implantierten Medikamentinfusionspumpe zu bestimmen.

4. Gerät nach einem der vorherigen Ansprüche, dadurch gekennzeichnet, daß das Übertragungshilfsmittel (20) eine Pumpe vom Kolbentyp umfaßt.

5. Gerät nach einem der vorherigen Ansprüche, dadurch gekennzeichnet, daß das Erzeugungshilfsmittel folgendes enthält: einen elektronischen Stethoskopkopf (22), der zur Aufnahme der vom Transporthilfsmittel erzeugten akustischen Signale eingebaut ist.

6. Gerät nach Anspruch 2 oder 3, dadurch gekennzeichnet, daß die Filterhilfsmittel (40) einen Hochpaßfilter zur wesentlichen Beseitigung von Körpergeräuschen aus den elektrischen Signalen umfaßt, wobei der Hochpaßfilter vorzugsweise zur Beseitigung von Signalen unter ca. 1 kHz ausgelegt ist.

7. Gerät nach Anspruch 6, dadurch gekennzeichnet, daß die Filterhilfsmittel (40) außerdem einen Tiefpaßfilter zur Beseitigung von Signalen über ca. 5 kHz aus den elektrischen Signalen umfassen.

8. Gerät nach einem der vorherigen Ansprüche, dadurch gekennzeichnet, daß das Verarbeitungshilfsmittel (26) zusätzlich folgendes umfaßt: einen Verstärker (42) zur Verstärkung besagter elektrischer Signale.

9. Gerät nach Anspruch 1, dadurch gekennzeichnet, daß die Logikhilfsmittel (54, 164) folgendes umfassen: Hilfsmittel zur Bestimmung einer auf die relative Amplitude der verarbeiteten elektrischen Signale bezogenen Kennziffer; und Hilfsmittel zum Vergleich der auf die relative Amplitude der verarbeiteten elektrischen Signale bezogenen Kennziffer mit einer Kennziffer von elektrischen Impulsbodensignalen, die sich auf ihre relative Amplitude beziehen, und zwar zur Bestimmung des Betriebszustands der implantierten Medikamentinfusionspumpe, wobei der erste vorbestimmte Wert wesentlich größer als die Kennziffer der elektrischen Impulsbodensignale ist, die sich auf ihre relative Amplitude beziehen, und der zweite vorbestimmte Wert wesentlich kleiner als die Kennziffer der elektrischen Impulsbodensignale ist, die sich auf ihre relative Amplitude beziehen.

10. Gerät nach Anspruch 9, dadurch gekennzeichnet, daß das Vergleichshilfsmittel zur Anzeige eines eingekapselten oder verstopften Katheters ausgelegt ist, wenn die relative Amplitude der verarbeiteten elektrischen Signale kleiner gleich dem zweiten vorbestimmten Wert ist.

11. Gerät nach Anspruch 10, dadurch gekennzeichnet, daß das Vergleichshilfsmittel ausgelegt ist, um einen eingekapselten oder verstopften Katheter anzuzeigen, wenn die relative Amplitude der verarbeiteten elektrischen Signale ungefähr ein fünfte der relativen Amplitude der elektrischen Impulsbodensignale oder weniger beträgt, wobei der zweite vorbestimmte Wert folglich ungefähr ein fünftel der Amplitude der elektrischen Impulsbodensignale beträgt.

12. Gerät nach Anspruch 11, bei dem das Vergleichshilfsmittel ausgelegt ist, um einen eingekapselten oder verstopften Katheter anzuzeigen, wenn die relative Amplitude der verarbeiteten elektrischen Signale kleiner als die relative Amplitude der elektrischen Impulsbodensignale oder gleich dem zweiten vorbestimmten Wert ist.

13. Gerät nach einem der vorherigen Ansprüche 10 bis 12, dadurch gekennzeichnet, daß das Vergleichshilfsmittel ausgelegt ist, um das Vorhandensein von Luft in der Flüssigleitung anzuzeigen, wenn die relative Amplitude der verarbeiteten elektrischen Signale größer als der erste vorbestimmte Wert ist.

14. Gerät nach Anspruch 13, dadurch gekennzeichnet, daß das Vergleichshilfsmittel ausgelegt ist, um anzuzeigen, daß die Stärke der verarbeiteten elektrischen Signale ungefähr doppelt so groß wie die relative Amplitude der elektrischen Impulsbodensignale oder höher ist, wobei der erste vorbestimmte Wert folglich ungefähr doppelt so groß wie die Amplitude der elektrischen Impulsbodensignale ist.

15. Gerät nach einem der Ansprüche 9 bis 14, dadurch gekennzeichnet, daß die Kennziffern der verarbeiteten elektrischen Signale und der auf ihre relativen Amplituden bezogenen elektrischen Impulsbodensignale folgendes enthalten: die Scheitelspannung im Zeitbereich sowohl der verarbeiteten elektrischen Signale als auch der elektrischen Impulsbodensignale; oder die mittlere Spannung im Zeitbereich jedes der verarbeiteten elektrischen Signale und elektrischen Impulsbodensignale; oder die mittlere Effektivspannung im Zeitbereich jedes der verarbeiteten elektrischen Signale und elektrischen Impulsbodensignale; oder das Integral im Zeitbereich jedes der verarbeiteten elektrischen Signale und elektrischen Impulsbodensignale; oder den Scheitelpegel im Frequenzbereich jedes der verarbeiteten elektrischen Signale und elektrischen Impulsbodensignale; oder den Pegel bei einer bestimmten Frequenz, zum Beispiel im Frequenzbereich jedes der verarbeiteten elektrischen Signale und elektrischen Impulsbodensignale.

16. Gerät nach einem der Ansprüche 9 bis 14, dadurch gekennzeichnet, daß die Kennziffern der verarbeiteten elektrischen Signale und auf ihre relativen Amplituden bezogenen elektrischen Impulsbodensignale folgendes enthalten: den Durchschnittspegel im Frequenzbereich jedes der verarbeiteten elektrischen Signale und besagten elektrischen Impulsbodensignale; oder den mittleren Effektivpegel im Frequenzbereich jedes der verarbeiteten elektrischen Signale und elektrischen Impulsbodensignale; oder das Integral im Frequenzbereich jedes der verarbeiteten elektrischen Signale und elektrischen Impulsbodensignale.

17. Gerät nach Anspruch 3, dadurch gekennzeichnet, daß das Logikhilfsmittel (54) einen teilweise eingekapselten oder verstopften Katheter anzeigt, wenn fallende Steilheitsmuster wiederholt auftreten.

18. Gerät nach einem der vorherigen Ansprüche, dadurch gekennzeichnet, daß das bei Feststellung einer Funktionsstörung des Systems bereitgestellte Informationssignal ein elektrisches "Kitzelsignal" mit niedrigem Pegel oder ein akustisches Signal umfaßt.

19. Gerät nach einem der vorherigen Ansprüche, gekennzeichnet durch Hilfsmittel zur Funktionshemmung des Transporthilfsmittels, wenn das Logikhilfsmittel das Auftreten einer Systemstörung feststellt.

20. Gerät nach Anspruch 3, dadurch gekennzeichnet, daß das Logikhilfsmittel (54) ein erstes Informationssignal bereitstellt, wenn die Steilheit größer gleich einem ersten vorbestimmten Wert ist, das Logikhilfsmittel ferner ein zweites Informationssignal bereitstellt, wenn die Steilheit kleiner gleich einem zweiten vorbestimmten Wert ist, wobei der zweite vorbestimmte Wert kleiner als der erste vorbestimmte Wert ist.

## Revendications

1. Dispositif pour administrer le médicament au patient, comprenant une pompe implantable à infusion du médicament, avec un réservoir pour contenir un médicament liquide et les moyens (20) pour transférer le médicament liquide du réservoir à un endroit à l'extérieur de la pompe implantable à infusion du médicament ; le dispositif comprend en outre des moyens (22) permettant de générer des signaux électriques proportionnels aux signaux acoustiques produits par la pompe à infusion du médicament implantée ; des moyens (26) pour traiter les signaux électriques afin de produire des signaux électriques traités ; des moyens (34) pour visualiser les signaux électriques traités afin de déterminer les caractéristiques liées à leur amplitude relative qui sont indicatives des conditions opérationnelles de la pompe à infusion du médicament implantée ; ainsi que des moyens logiques ( 54,164) disposés pour utiliser les caractéristiques liées à l'amplitude relative des signaux électriques traités pour déterminer les conditions opérationnelles de la pompe à infusion du médicament implantée, les moyens logiques comprenant des moyens pour fournir un signal informationnel si le moyen logique détecte l'existence d'un fonctionnement défectueux du système ; caractérisé par le fait que les moyens (22) pour produire les signaux électriques sont des moyens non invasifs de génération de signaux électriques, les moyens logiques (54,164) produisant un premier signal informationnel si les signaux électriques traités sont plus grands que, ou égaux à une première valeur prédéterminée, les moyens logiques fournissant un second signal informationnel si les signaux traités sont plus petits que, ou égaux à une seconde valeur prédéterminée étant plus petite que la première valeur prédéterminée.

2. Dispositif selon la revendication 1, caractérisé par le fait que les moyens de traitement (26) comprennent des moyens de filtration (40) qui filtrent les signaux électriques afin de produire des signaux électriques filtrés ; et les moyens logiques (54) sont disposés afin de comparer les caractéristiques liées à l'amplitude des signaux électriques filtrés avec les caractéristiques des signaux électriques de la ligne de base liées à leur amplitude relative afin de déterminer l'état opérationnel de la pompe à infusion du médicament implantée.

3. Dispositif selon la revendication 1, caractérisé par le fait que les moyens de traitement (26) comprennent des moyens de filtration (40) qui filtrent les signaux électriques afin de fournir des signaux électriques filtrés ; et l'affichage (34) ou moyens logiques sont disposés pour utiliser les caractéristiques liées à la courbe de leur amplitude relative des signaux filtrés durant des cycles de pompage répétitifs et peu espacés afin de déterminer l'état opérationnel de la pompe à infusion du médicament implantée.

4. Dispositif selon l'une quelconque des revendications précédentes, caractérisé par le fait que les moyens de transfert comprennent: une pompe du genre à piston.

5. Dispositif selon l'une quelconque des revendications précédentes, caractérisé par le fait que les moyens de production comprennent : une tête de stéthoscope électronique (22) conçue pour détecter les signaux acoustiques produits par les moyens de transfert.

6. Dispositif selon les revendications 2 ou 3, caractérisé par le fait que les moyens de filtration (40) comprennent un filtre passe-haut pour éliminer de façon substantielle les bruits corporels dans les signaux électriques, le filtre passe-haut étant si possible réglé pour éliminer les signaux approximativement inférieurs à 1 KHz.

7. Dispositif selon la revendication 6, caractérisé par le fait que le moyen filtrant (40) comprend également un filtre passe-bas afin d'éliminer des signaux électriques, les signaux au-dessus de 5 KHz approximativement.

8. Dispositif selon l'une quelconque des revendications précédentes, caractérisé par le fait que les moyens de traitement (26) comprennent en outre : un amplificateur (42) pour amplifier les signaux électriques mentionnés.

9. Dispositif selon la revendication 1, caractérisé par le fait que le moyen logique (54,164) comprend : des moyens pour déterminer une caractéristique liée à l'amplitude relative des signaux électriques traités ; et des moyens pour comparer les caractéristiques liées à l'amplitude relative des signaux électriques traités avec une caractéristique de signaux électriques de ligne de base liée à leur amplitude relative pour déterminer les conditions opérationnelles de la pompe à infusion du médicament implantée, la première valeur prédéterminée étant considérablement plus grande que la caractéristique des signaux électriques de ligne de base liée à leur amplitude relative, la seconde valeur prédéterminée étant considérablement plus petite que la caractéristique des signaux électriques de la ligne de base liée à leur amplitude relative.

10. Dispositif selon la revendication 9, caractérisé par le fait que le moyen de comparaison est disposé afin d'indiquer : un cathéter encapsulé ou occlus quand l'amplitude relative des signaux électriques traités est plus petite que, ou égale à la deuxième valeur prédéterminée.

11. Dispositif selon la revendication 10, caractérisé par le fait que le moyen de comparaison est disposé de façon à indiquer la présence d'un cathéter encapsulé ou occlus quand l'amplitude relative des signaux électriques traités est approximativement un cinquième ou moins, de l'amplitude relative des signaux électriques de ligne de base, la deuxième valeur prédéterminée étant ainsi approximativement un cinquième de l'amplitude des signaux électriques de la ligne de base.

12. Dispositif selon la revendication 11, dans lequel le moyen de comparaison est disposé de façon à indiquer un cathéter encapsulé ou occlus quand l'amplitude relative des signaux électriques traités est plus petite que l'amplitude relative du signal électrique de la ligne de base, ou égale à la deuxième valeur prédéterminée.

13. Dispositif selon l'une quelconque des revendications 10 à 12, caractérisé par le fait que le moyen de comparaison est disposé de façon à indiquer la présence d'air dans la ligne du fluide quand l'amplitude relative des signaux électriques traités est plus grande que la première valeur prédéterminée.

14. Dispositif selon la revendication 13, caractérisé par le fait que le moyen de comparaison est disposé de façon à indiquer que la présence des signaux électriques traités est approximativement égale à deux fois ou plus, l'amplitude relative des signaux électriques de la ligne de base, la première valeur prédéterminée étant ainsi approximativement égale à deux fois l'amplitude des signaux électriques de la ligne de base.

15. Dispositif selon l'une quelconque des revendications de 9 à 14, caractérisé par le fait que les caractéristiques des signaux électriques traités ainsi que les signaux électriques de la ligne de base liées à leurs amplitudes relatives comprennent : le voltage de crête dans le domaine temporel de chacun des signaux électriques traités ainsi que des signaux électriques de la ligne de base ; ou le voltage moyen dans le domaine temporel de chacun des signaux électriques traités et les signaux de la ligne de base ; ou le voltage moyen des RMS dans le domaine temporel de chacun des signaux électriques traités et les signaux électriques de la ligne de base ; ou l'intégral dans le domaine temporel de chacun des signaux électriques traités ainsi que des signaux électriques de la ligne de base ; ou le niveau de crête dans le domaine fréquentiel de chacun des signaux électriques traités ainsi que des signaux électriques de la ligne de base ; ou le niveau à une fréquence particulière, par exemple, dans le domaine fréquentiel de chacun des signaux électriques traités ou des signaux électriques de la ligne de base.

16. Dispositif selon l'une quelconque des revendications 9 à 14, caractérisé par le fait que les caractéristiques des signaux électriques traités ainsi que les signaux électriques de la ligne de base liées à leurs amplitudes relatives comprennent : le niveau moyen dans le domaine fréquentiel de chacun des signaux électriques traités et les dits signaux électriques de base; ou le niveau moyen des RMS dans le domaine fréquentiel de chacun des signaux électriques traités ainsi que les signaux électriques de la ligne de base; ou l'intégral dans le domaine fréquentiel de chacun des signaux électriques traités ainsi que les signaux électriques de la ligne de base.

17. Dispositif selon la revendication 3, caractérisé par le fait que le moyen logique (54) indique la présence d'un cathéter partiellement encapsulé ou occlus quand se présentent des tracés en pente descendante répétés.

18. Dispositif selon l'une quelconque des revendications précédentes, caractérisé par le fait que le signal informationnel envoyé lorsque se présente un mauvais fonctionnement du système, comprend un signal électrique causant un léger 〈〈chatouillement〉〉 ou un signal audible.

19. Dispositif selon l'une quelconque des revendications précédentes, caractérisé par un moyen pouvant inhiber la mise en opération du moyen de transfert si le moyen logique découvre l'existence d'un mauvais fonctionnement du système.

20. Dispositif selon la revendication 3, caractérisé par le fait que le moyen logique (54) donne un premier signal informationnel si la pente est plus grande que, ou égale à, une première valeur prédéterminée, le moyen logique donnant un deuxième signal informationnel si la pente est plus petite que, ou égale à, une seconde valeur prédéterminée, la seconde valeur prédéterminée étant plus petite que la première valeur prédéterminée.
